# EUROPEAN PATENT SPECIFICATION

(11) **EP 3 003 980 B1**
(45) Date of publication and mention of the grant of the patent: **08.04.2020**
(21) Application number: 14732402.4
(22) Date of filing: 22.05.2014
(51) Int. Cl.: C07C 273/16, C01C 1/242, C01C 1/12

(54) **METHOD FOR RECOVERING AMMONIUM SULPHATE FROM A UREA PLANT GAS STREAM**
VERFAHREN ZUR RÜCKGEWINNUNG VON AMMONIUMSULFAT AUS EINEM GASSTROM EINER HARNSTOFFANLAGE
PROCÉDÉ DE RÉCUPÉRATION DE SULFATE D'AMMONIUM DANS UN FLUX GAZEUX D'USINE D'URÉE

(30) Priority: 24.05.2013 IT MI20130847
(43) Date of publication of application: 13.04.2016
(73) Proprietor: Saipem S.p.A., 20097 San Donato Milanese (Milano) (IT)
(72) Inventor: BRUNO, Lorenzo, I-20097 San Donato Milanese (IT); GIANAZZA, Alessandro, I-20097 San Donato Milanese (IT); CARLESSI, Lino, I-24044 Dalmine (IT)
(74) Representative: Cernuzzi, Daniele
(86) International application number: PCT/IB2014/061626
(87) International publication number: WO 2014/188371

(56) References cited:
- EP-A1- 2 192 099
- WO-A1-2006/004424
- WO-A2-01/51429
- US-A- 3 785 796

## Description

### TECHNICAL FIELD

The present invention relates to a method for recovering ammonium sulphate from a gas stream from a urea (i.e. urea production) plant.

More specifically, the present invention relates to a method for recovering ammonium sulphate from a gas stream produced in a solidification (granulating or prilling) unit of the urea plant.

### BACKGROUND ART

As is known, industrial-scale urea production processes are based on a high-temperature, high-pressure reaction of carbon dioxide and ammonia to form ammonium carbamate, and a subsequent decomposition reaction of the ammonium carbamate to form urea and water.

In a common-type urea production plant (urea plant), these processes are normally carried out in a urea synthesis reactor; the water and urea solution produced in the synthesis reactor is then concentrated gradually, and the non-converted reactants recovered in one or more recovery sections, e.g. a high-pressure section, medium-pressure section, and low-pressure section; and, finally, the urea is solidified in a solidification section normally comprising a granulator or prilling tower.

More specifically, the urea from the synthesis reactor is concentrated gradually to roughly over 95% (normally 96-99.7%) by weight before being fed to the solidification section (granulator or prilling tower) to obtain the commercial end product in the form of granulated or prilled urea (urea granules or prills).

The urea is normally fed to the granulator or prilling tower in the liquid state, and is solidified by a stream of ambient air.

The solidifying air contains ammonia released from the urea during the cooling and solidification process, and should therefore be treated, to remove its ammonia content, before being released into the atmosphere.

In one known method of removing ammonia from the cooling air, the air is fed into a scrubber where it is brought into contact with a sulphuric acid solution and produces as a by-product a solution containing up to 40% ammonium sulphate.

As a reaction by-product, ammonium sulphate is hygroscopic, of little commercial value, and difficult to process chemically and physically.

Since for some crops, however, ammonium sulphate, far from being a contaminant, is even beneficial, ammonium sulphate recovered from urea plants may be used to advantage for producing urea-based fertilizers.

One process for recovering ammonium sulphate from urea plants is described in EP2192099-A1. In this process, an aqueous solution of urea, water, and ammonium sulphate is produced and, after being concentrated in an evaporator usually to at most 12%, is forwarded to a granulating stage. Since this process employs a granulator, which, as is known, also operates in the presence of significant amounts of water, eliminating all or almost all the water from the solutions fed to the granulator is neither necessary nor provided for.

In urea plants with prilling tower solidification sections, on the other hand, to avoid impairing the quality of the end product, the prilling towers can only be supplied with solutions containing small traces (roughly 0.2-0.3% by weight) of water.

In plants such as these, processes of the type described above are impossible to use, on the grounds that eliminating water from ammonium sulphate makes it practically unusable.

As stated, ammonium sulphate is extremely difficult to process chemically and physically, especially at the water-removing and melt stages.

The water and ammonium sulphate solution, in fact, begins boiling at a concentration of about 51% by weight and a temperature of 108.5 °C. At this concentration, crystallization commences, and the crystalline substance is difficult to process and mix with urea.

Pure crystallized ammonium sulphate is also impossible to melt to inject it using pumps. In fact, before it melts, ammonium sulphate begins decomposing at a temperature of 230-280°C.

EP2192098A1 discloses a urea granulation process (and an apparatus suitable for operating that process) integrating a method for reducing ammonia emissions by scrubbing the off-gas and recovering the scrubber bleed and integrating it into the granulation process so that ammonium salts are completely contained by the process.

WO2006/004424A1 discloses a method for the production of solid urea ammonium sulphate (UAS) fertilizers from sulphuric acid, ammonia and urea, wherein free ammonia and/or as carbamate to be decomposed from urea production, is reacted with sulphuric acid without substantially decomposing urea in the process stream, where after urea and ammonium sulphate (AS) is mixed and particulated.

WO01/51429A2 discloses a method for producing a fertilizer containing ammonium sulfate and urea. An aqueous urea solution is reacted with sulfuric acid and, optionally, with ammonia in order to form a urea-ammonium sulfate solution or suspension in quantities that result in a predetermined weight ratio of ammonium sulfate to urea, whereby CO2 and ammonia are returned to the urea synthesis. Afterwards, the urea-ammonium sulfate solution or suspension is subjected to a subsequent processing in order to produce solid or liquid fertilizers.

US3785796A describes a process of producing urea granules from urea and ammonium sulphate mixtures containing a high percentage (15-70% by weight) of ammonium sulphate, and, among other things, the conditions in which urea and ammonium sulphate form a eutectic. This process, however, is not applicable to recovering ammonium sulphate from urea plants.

### DISCLOSURE OF INVENTION

It is an object of the present invention to provide a method for recovering ammonium sulphate from a gas stream produced in a urea plant, and in particular by a solidification unit of the plant, which enable ammonium sulphate to be recovered effectively and relatively cheaply and easily also from urea plants employing prilling towers (though applicable to granulators, too).

According to the present invention, there is provided a method for recovering ammonium sulphate from a gas stream produced in a urea plant, and in particular by a solidification unit of the plant, as defined in the accompanying Claim 1.

Additional preferred characteristics are indicated in the dependent Claims.

The method according to the invention enable the ammonium sulphate by-product, of little value, to be recycled to produce, relatively cheaply and easily, an end product containing ammonium sulphate and a higher percentage - roughly 99.5% - of urea than known solutions.

The invention may also be used in both existing and new urea plants employing prilling or granulating units.

### BRIEF DESCRIPTION OF DRAWINGS

A number of embodiments of the present invention will be described by way of example with reference to the attached drawings, in which :
Figure 1 shows a highly simplified, schematic block diagram of a urea production plant equipped with an ammonium sulphate recovery system operating in accordance with the method of the invention;
Figure 2 shows a schematic of a first embodiment of an ammonium sulphate recovery system which can be used in the method according to the invention;
Figure 3 shows a schematic of a variation of the Figure 2 embodiment;
Figure 4 shows a schematic of a second embodiment of an ammonium sulphate recovery system which can be used in the method according to the invention;
Figure 5 shows a graph of significant chemical and physical parameters of urea and ammonium sulphate solution mixtures and the preferred operating range according to the invention.

### BEST MODE FOR CARRYING OUT THE INVENTION

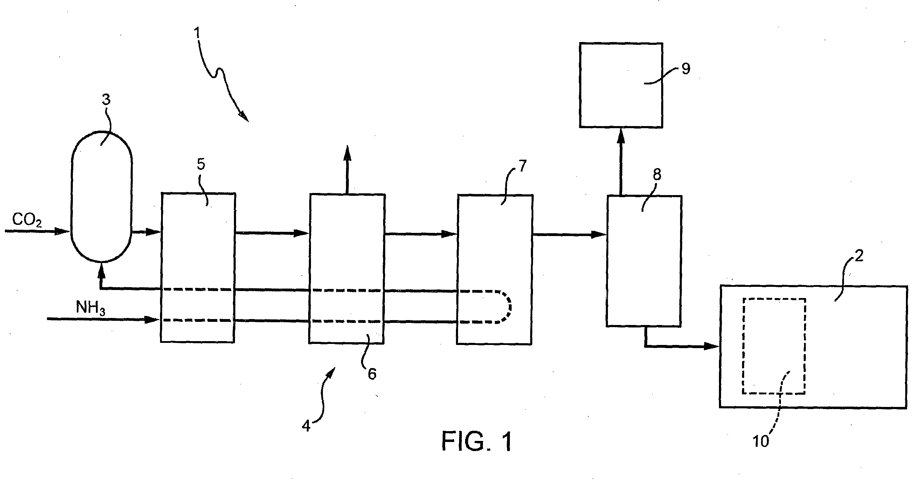

Figure 1 shows a schematic of a urea (i.e. urea production) plant 1 equipped with a recovery system 2 for recovering ammonium sulphate from a gas stream produced in urea plant 1, and in particular from a gas stream from a solidification unit of urea plant 1.

Urea plant 1 substantially comprises : a urea synthesis reactor 3 where urea is synthesized from ammonia and carbon dioxide; recovery sections 4 - in particular, a high-pressure section 5, a medium-pressure section 6, and a low-pressure section 7 - where the urea solution produced in synthesis reactor 3 is gradually concentrated, the water and non-reacting ammonia and carbon dioxide are removed, and the recovered components are recirculated; a vacuum section 8 equipped with a vacuum system and connected to a drain water processing section 9; and at least one solidification unit 10, e.g. a granulating or prilling unit, forming part of or connected functionally to recovery system 2, and where the urea produced is formed into granules or prills.

For the sake of simplicity, Figure 1 does not show the component parts of each section, or in detail the lines conducting and recirculating the various streams from one section to another. Figure 1 simply shows schematically the reactant feed lines to urea plant 1, and the operating connections between the sections.

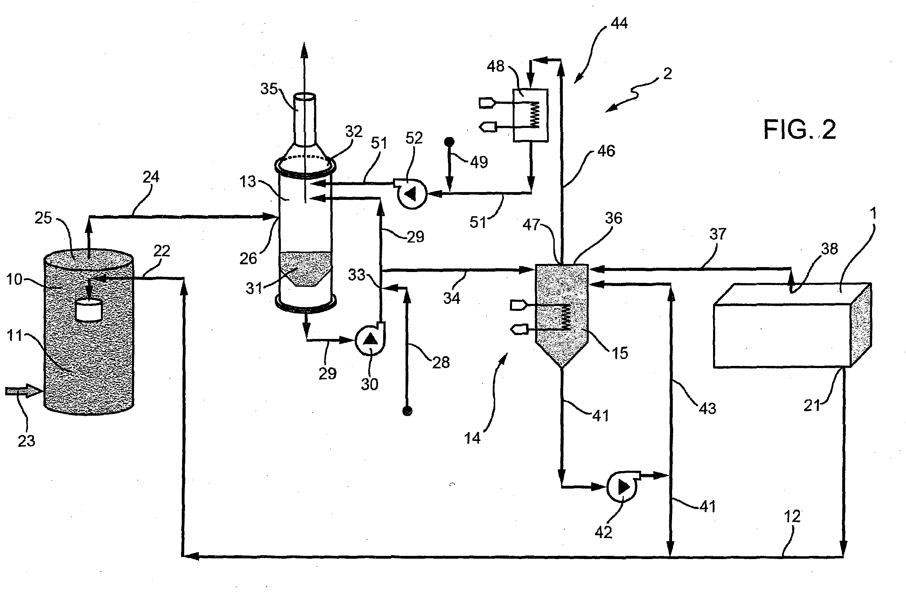

In the Figure 2 embodiment, solidification unit 10 comprises a prilling tower 11, connected to urea plant 1 by a main feed line 12 for drawing a flow of high-concentration urea melt from urea plant 1 (by which is meant a typical urea plant product with a concentration of over roughly 95%, and roughly of about 96.0 to 99.7 by weight; high-concentration urea melt normally has a concentration of about 96% by weight in granulator urea plants, and about 99.7% in prilling unit urea plants).

Recovery system 2 comprises a scrubber 13, connected to, and for treating an exhaust gas stream from, prilling tower 11; and a mixing circuit 14 equipped with an evaporator 15 and connecting scrubber 13 to main feed line 12 to supply prilling tower 11, together with the high-concentration urea melt, with a urea and ammonium sulphate mixture containing traces of water, and more specifically a urea and ammonium sulphate eutectic.

Main feed line 12 connects a main outlet 21 of urea plant 1 to an inlet 22 of prilling tower 11, and draws and feeds high-concentration urea melt from urea plant 1 to prilling tower 11.

Prilling tower 11 is fed with a stream of cooling air, shown schematically by arrow 23 in Figure 2, which flows into prilling tower 11 through air inlet openings to solidify the urea melt into urea prills.

The gas stream from prilling tower 11, substantially composed of the cooling air used in prilling tower 11 and containing ammonia (released by the urea as it cools and solidifies) is fed to scrubber 13 along a gas line 24 connecting the top 25 of prilling tower 11 to a gas inlet 26 on scrubber 13.

Scrubber 13 serves to remove ammonia from the gas stream from prilling tower 11; the ammonia is removed inside scrubber 13 by reaction with sulphuric acid to form ammonium sulphate, which is removed from scrubber 13 in an aqueous solution.

The sulphuric acid used (e.g. with a concentration of 93-98%) is fed to scrubber 13 along an acid feed line 28.

Scrubber 13 has an outflow line 29 fitted with a recirculating pump 30, and which connects the bottom 31 of scrubber 13 to the top 32 of scrubber 13 to recirculate an outflow stream containing the water and ammonium sulphate solution produced in scrubber 13. Outflow line 29 is connected to acid feed line 28 at a junction 33, so that sulphuric acid is fed into scrubber 13 mixed with part of the outflow (ammonium sulphate solution) from scrubber 13. Recirculating pump 30 is located, for example, along outflow line 29, upstream from junction 33.

Scrubber 13 produces an ammonium sulphate solution with a concentration of up to roughly 40% by weight, which is drawn from the bottom 31 of scrubber 13 along outflow line 29 and, after fresh sulphuric acid is added, is recirculated partly back into scrubber 13, and partly to evaporator 15 along a draw-off line 34.

Scrubber 13 has a gas outlet 35, from which the clean, i.e. ammonia-free, gases flow out.

Mixing circuit 14 comprises : evaporator 15; draw-off line 34, which connects outflow line 29 of scrubber 13 to the top 36 of evaporator 15; a secondary feed line 37, which connects an auxiliary outlet 38 of urea plant 1 to the top 36 of evaporator 15; a mixture outflow line 41 fitted with a circulating pump 42, and which extends from evaporator 15 to main feed line 12; and a recirculating line 43 connecting mixture outflow line 41 to the top 36 of evaporator 15.

Draw-off line 34 branches off from outflow line 29 of scrubber 13 downstream from junction 33, i.e. downstream from where outflow line 29 connects to acid feed line 28, and supplies evaporator 15 with a water and ammonium sulphate solution with a concentration of up to 40% by weight.

Secondary feed line 37 draws a low-concentration urea melt solution from urea plant 1 (by which is meant a water and urea melt solution with a concentration of less than roughly 90% by weight, and of roughly 85%) and feeds it to evaporator 15.

Evaporator 15 is preferably a vacuum evaporator, which is heated by low-pressure vapour, advantageously from urea plant 1 and drawn, for example, from high-pressure section 5.

Evaporator 15 is thus fed with the low-concentration (e.g. roughly 85% by weight) urea solution from urea plant 1 and with the water and ammonium sulphate solution, with a concentration of up to 40% by weight, from scrubber 13.

Both the solutions (low-concentration urea solution and ammonium sulphate solution) are concentrated in evaporator 15 to separate a vapour phase by means of the hot vapour fed preferably into the mantle of evaporator 15, and to form a urea and ammonium sulphate mixture containing only residual traces of water (water content less than 1%, preferably less than 0.5%, and more preferably less than 0.3% by weight) and therefore compatible with supply to prilling tower 11.

More specifically, the ammonium sulphate solution, with a concentration of up to 40% by weight, produced in scrubber 13 is mixed with a urea solution, with a concentration of roughly 85% by weight, from vacuum section 8 of urea plant 1 to form, once the water is evaporated, a urea-ammonium sulphate eutectic melt.

As is known, a eutectic is a mixture of substances having a lower melting point than each of its component substances.

To avoid decomposition, instability, and crystallization problems, the mixture (eutectic) must have an ammonium sulphate concentration of less than 60%.

This prevents the formation of crystallized ammonium sulphate, in that, with a 30-60% by weight ammonium sulphate concentration, the eutectic melts within a temperature range of 121-150°C.

More specifically, 0.3-0.5 kg of 85% by weight urea solution is added per kilo of water and ammonium sulphate solution to form a mixture containing 15-30% urea, roughly 30% ammonium sulphate, and the remainder of water. The water is subsequently eliminated in evaporator 15 to form the urea-ammonium sulphate eutectic.

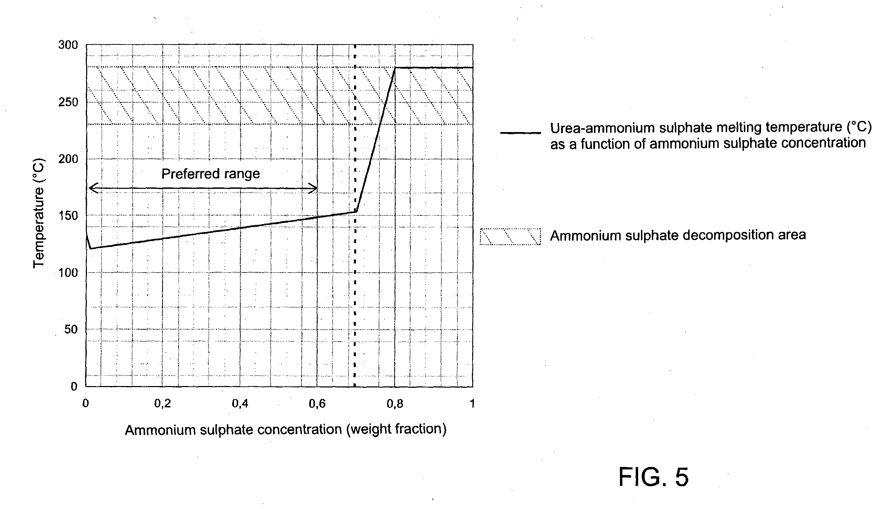

A few significant chemical and physical parameters of the urea-ammonium sulphate eutectic are shown in Figure 5, and more specifically :
- the urea-ammonium sulphate eutectic melting point profile;
- the ammonium sulphate decomposition area;
- the preferred composition range according to the invention.

To prevent any ammonia in the 85% urea solution from urea plant 1 from being released in evaporator 15, the ammonium sulphate solution is drawn off downstream from the point (i.e. junction 33) at which sulphuric acid is injected into scrubber 13. This way, any surplus sulphuric acid combines with surplus ammonia in the 85% urea solution to form ammonium sulphate and prevent the ammonia from ending up in the vapour phase released inside evaporator 15.

In other words, evaporator 15 produces a urea-ammonium sulphate eutectic melt with traces of water and at a temperature of roughly 135°C (eutectic containing 30-50% urea and with a melting temperature of 121-150°C).

The urea-ammonium sulphate eutectic melt (at roughly 135°C) from evaporator 15 is fed along mixture outflow line 41 to main feed line 12 to mix with the high-concentration 96-99.7% urea melt from urea plant 1 (also at roughly 135°C). As stated, the high-concentration urea melt has a concentration of about 96% by weight in plants employing granulators, and of about 99.7% by weight in plants employing prilling units.

The high-concentration urea flow rate is roughly 100-200 times that of the urea-ammonium sulphate eutectic from evaporator 15.

The final mixture contains less than 0.5% by weight of ammonium sulphate, and is fed to prilling tower 11.

Evaporator 15 for eliminating water from the mixture is preferably a vacuum evaporator, to prevent the formation of biuret possibly resulting from urea degradation and which is an undesired by-contaminant.

The vapour from the top 36 of evaporator 15 is fed to a vacuum system 44.

More specifically, a vapour line 46 connects a vapour outlet 47 of evaporator 15 to a condenser 48 where the vapour phase separated in evaporator 15 is condensed, e.g. by heat exchange with cold water, to form a condensate. The condensate produced in condenser 48 is fed, possibly together with outside water supplied by a water feed line 49, to the top 32 of scrubber 13 along a condensate line 51 fitted with a pump 52 and connecting condenser 48 to scrubber 13.

To implement the method according to the invention, recovery system 2 operates as follows.

The gas stream from solidification unit 10 (in the example shown, prilling tower 11) is treated in scrubber 13, connected to solidification unit 10, to remove ammonia from the gas stream and form an ammonium sulphate solution.

In scrubber 13, the ammonia in the gas stream from solidification unit 10 is removed by reaction with sulphuric acid to form a water and ammonium sulphate solution, which is fed from scrubber 13 to evaporator 15.

Scrubber 13 is supplied with sulphuric acid; the ammonium sulphate solution from scrubber 13 is partly recirculated back into the top of scrubber 13, after fresh sulphuric acid is added, and partly fed to evaporator 15 to mix with the low-concentration 85% by weight urea solution produced in urea plant 1.

More specifically, the ammonium sulphate solution produced in scrubber 13 is drawn from scrubber 13, with the addition of sulphuric acid, and is mixed in evaporator 15 with the low-concentration 85% by weight urea solution produced in urea plant 1; and water is removed inside evaporator 15 to form the urea-ammonium sulphate eutectic.

The urea-ammonium sulphate eutectic, mixed with the high-concentration urea melt produced in urea plant 1, is fed to solidification unit 10.

As stated, the low-concentration urea solution fed to evaporator 15 has a lower concentration than the high-concentration urea melt mixed with the eutectic.

More specifically, the low-concentration urea melt solution has a concentration of less than 95% by weight, preferably of less than 90% by weight, and roughly of about 85% by weight; and the high-concentration urea melt has a typical concentration of over roughly 95%, and of roughly 96.0 to 99.7% by weight.

Preferably, the water and ammonium sulphate solution fed from scrubber 13 to evaporator 15 has a concentration of up to 40% by weight, and the urea solution has a concentration of roughly 85% by weight.

In evaporator 15, the ammonium sulphate solution from scrubber 13 and the low-concentration urea solution are mixed and concentrated to separate a vapour phase and form the urea-ammonium sulphate eutectic.

The eutectic has an ammonium sulphate concentration of less than 60% by weight.

Part of the eutectic from evaporator 15 is recirculated back into the top of evaporator 15, and the rest is mixed with the high-concentration urea melt. The high-concentration urea melt flow rate is roughly 100-200 times that of the urea-ammonium sulphate eutectic from evaporator 15.

The resulting final mixture contains less than 0.5% by weight of ammonium sulphate.

The vapour phase separated in evaporator 15 is fed to condenser 48 where it is condensed to form a condensate, which is fed, possibly together with outside water, to scrubber 13.

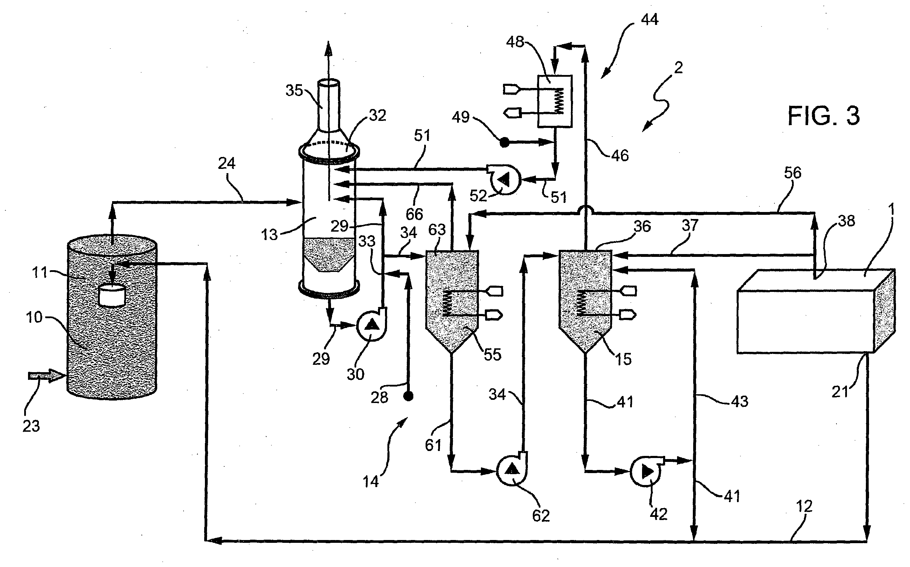

Figure 3, in which any details similar or identical to those already described are indicated using the same reference numbers, shows a variation of recovery system 2 described above, and which is particularly advantageous when working with high-flow-rate, highly diluted ammonium sulphate solutions from scrubber 13.

In this case, recovery system 2 comprises a pre-concentrator 55 - more specifically, an atmospheric pre-concentrator - located upstream from evaporator 15 along draw-off line 34, to treat and pre-concentrate the ammonium sulphate solution from scrubber 13 before it is fed to evaporator 15. Pre-concentrator 55 is heated, for example, by low-pressure vapour drawn from high-pressure section 5.

Secondary feed line 37 connecting urea plant 1 to evaporator 15, to supply evaporator 15 with low-concentration, 85% by weight urea solution, has a branch 56 for feeding part of the low-concentration, 85% by weight urea solution to pre-concentrator 55.

Pre-concentrator 55 has an outflow line 61 equipped with a circulating pump 62, and which extends from pre-concentrator 55 to the top 36 of evaporator 15 to define a portion of draw-off line 34.

Draw-off line 34 uses circulating pump 30 and connects outflow line 29 of scrubber 13 (downstream from junction 33, i.e. from where outflow line 29 connects to acid feed line 28 and downstream from circulating pump 30) to pre-concentrator 55, to feed the latter with the water and ammonium sulphate solution with a concentration of up to 40% by weight.

Pre-concentrator 55 concentrates and mixes the above solution with the low-concentration (roughly 85% by weight) urea solution to form a concentrated aqueous solution containing, for example, roughly 40-50% by weight of ammonium sulphate, and which is fed to evaporator 15 to remove the water and form the eutectic described previously.

The vapour from the top 63 of pre-concentrator 55 is fed along a vapour line 66 to the top 32 of scrubber 13.

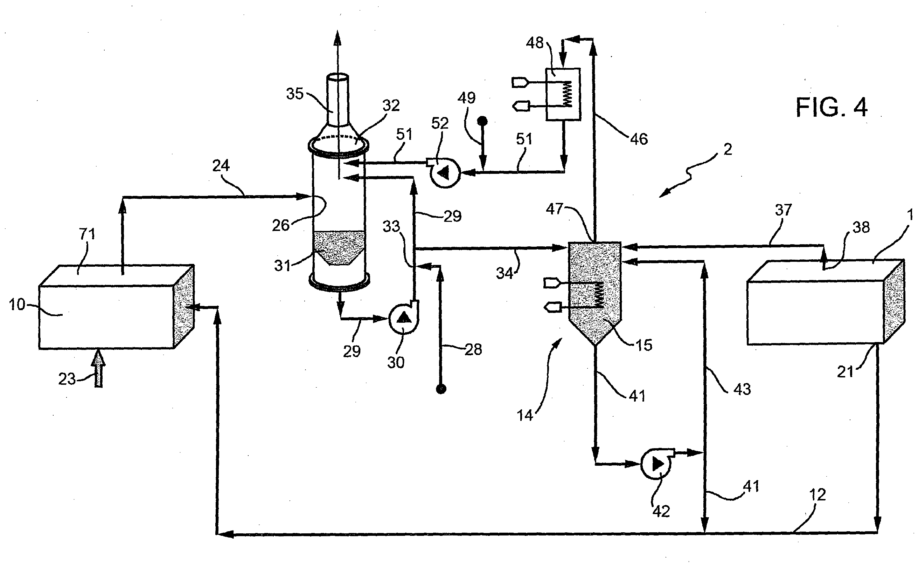

In the Figure 4 embodiment, in which any details similar or identical to those already described are indicated using the same reference numbers, solidification unit 10 of urea plant 1 is a granulating unit, comprising a granulator 71.

Granulator 71 also operates with a stream of cooling air, shown schematically by arrow 23 in Figure 4, and which, after cooling the urea, is fed along gas line 24 to scrubber 13 to remove ammonia.

Recovery system 2 is identical to the one described with reference to Figures 2 and 3, and requires no particular alterations to adapt to the use of granulator 71.

## Claims

1. A method of recovering ammonium sulphate from a gas stream produced in a urea plant (1), and in particular by a solidification unit (10) of the urea plant, comprising the steps of :
- treating a gas stream from a solidification unit (10) in a scrubber (13) connected to the solidification unit (10), to remove ammonia from said gas stream by reaction with sulphuric acid with formation of a water and ammonium sulphate solution;
- drawing the ammonium sulphate solution off from the scrubber (13);
- mixing the ammonium sulphate solution with a low-concentration urea solution produced in the urea plant (1), and removing water in an evaporator (15), to form a urea-ammonium sulphate eutectic; the eutectic having a 30-60% by weight ammonium sulphate concentration, a melt temperature of 121-150°C and a water content less than 1%;
- recirculating part of said eutectic from the evaporator (15) to the top (36) of the evaporator (15), and mixing the rest with high-concentration urea melt produced by the urea plant (1);
- feeding said eutectic, mixed with high-concentration urea melt produced by the urea plant (1), to the solidification unit (10);
wherein the low-concentration urea solution fed to the evaporator (15) has a lower concentration than the high-concentration urea melt mixed with the eutectic; and wherein the ammonium sulphate solution formed in the scrubber (13) is fed to the evaporator (15) where the urea-ammonium sulphate eutectic is formed; the high-concentration urea melt having a flow rate of 100-200 times that of the urea-ammonium sulphate eutectic from the evaporator (15) and the final mixture resulting from mixing the high-concentration urea melt and the urea-ammonium sulphate eutectic contains less than 0.5% by weight of ammonium sulphate;
and wherein the low-concentration urea solution has a concentration of less than 95% by weight.

2. A method as claimed in Claim 1, wherein the ammonium sulphate solution drawn from the scrubber (13) is supplemented with sulphuric acid before being fed to the evaporator (15).

3. A method as claimed in Claim 1 or 2, wherein the ammonium sulphate solution fed to the evaporator (15) has a concentration of up to roughly 40% by weight.

4. A method as claimed in one of the foregoing Claims, wherein the low-concentration urea solution has a concentration of less than 90% by weight.

5. A method as claimed in one of the foregoing Claims, comprising a step of concentrating the ammonium sulphate solution formed in the scrubber (13) and the low-concentration urea solution, with separation of a vapour phase and formation of the urea-ammonium sulphate eutectic.

6. A method as claimed in one of the foregoing Claims, wherein the ammonium sulphate solution from the scrubber (13) has a concentration of up to roughly 40% by weight, and the low-concentration urea solution produced in the urea plant (1) has a concentration of roughly 85% by weight.

7. A method as claimed in one of the foregoing Claims, wherein the eutectic has an ammonium sulphate concentration of less than 60% by weight.

8. A method as claimed in one of the foregoing Claims, wherein the solidification unit (10) comprises a prilling tower (11), or a granulator (71).

9. A method as claimed in one of the foregoing Claims, comprising the steps of feeding sulphuric acid to the scrubber (13); and recirculating to the scrubber (13) an outflow containing the ammonium sulphate solution produced in the scrubber (13).

10. A method as claimed in Claim 9, wherein part of the ammonium sulphate solution from the scrubber (13) is recirculated back into the scrubber (13), after the addition of fresh sulphuric acid, and part is fed to the evaporator (15).

11. A method as claimed in one of the foregoing Claims, wherein the evaporator (15) is a vacuum evaporator.

12. A method as claimed in one of the foregoing Claims, comprising the steps of : feeding a vapour phase, separated in the evaporator (15), to a condenser (48); and condensing the vapour phase to form a condensate which, possibly with the addition of outside water, is fed to the scrubber (13).

13. A method as claimed in one of the foregoing Claims, comprising a step of pre-concentrating the ammonium sulphate solution from the scrubber (13) before it is fed to the evaporator (15).

14. A method as claimed in Claim 13, comprising a step of mixing the concentrated ammonium sulphate solution with a first part of the low-concentration urea solution, to form a concentrated aqueous solution containing roughly 40-50% by weight of ammonium sulphate, and which is fed to the evaporator (15) to remove the water and form the urea-ammonium sulphate eutectic.

## Patentansprüche

1. Verfahren zur Rückgewinnung von Ammoniumsulfat aus einem Gasstrom, der in einer Harnstoffanlage (1) und insbesondere durch eine Verfestigungseinheit (10) der Harnstoffanlage erzeugt wird, umfassend die folgenden Schritte:
- Behandlung eines Gasstroms aus einer Verfestigungseinheit (10) in einem Wäscher (13), der mit der Verfestigungseinheit (10) verbunden ist, um Ammoniak aus dem genannten Gasdampf durch Reaktion mit Schwefelsäure unter Bildung einer Wasser- und Ammoniumsulfatlösung zu entfernen;
- Abziehen der Ammoniumsulfatlösung aus dem Wäscher (13);
- Mischen der Ammoniumsulfatlösung mit einer in der Harnstoffanlage (1) hergestellten Harnstofflösung niedriger Konzentration und Entfernen des Wassers in einem Verdampfer (15), um ein Harnstoff-Ammoniumsulfat-Eutektikum zu bilden; wobei das Eutektikum eine Ammoniumsulfatkonzentration von 30 bis 60 Gew.-%, eine Schmelztemperatur von 121 bis 150°C und einen Wassergehalt von weniger als 1% aufweist;
- Rückführung eines Teils des Eutektikums vom Verdampfer (15) zum oberen Teil (36) des Verdampfers (15) und Mischen des Restes mit hochkonzentrierter Harnstoffschmelze, die von der Harnstoffanlage (1) erzeugt wird;
- Einspeisung des Eutektikums, gemischt mit der von der Harnstoffanlage (1) erzeugten hochkonzentrierten Harnstoffschmelze, in die Verfestigungseinheit (10); wobei die dem Verdampfer (15) zugeführte niedrig konzentrierte Harnstofflösung eine geringere Konzentration als die mit dem Eutektikum vermischte hoch konzentrierte Harnstoffschmelze aufweist; und wobei die in dem Wäscher (13) gebildete Ammoniumsulfatlösung dem Verdampfer (15) zugeführt wird, wo das Harnstoff-Ammoniumsulfat-Eutektikum gebildet wird; wobei die hochkonzentrierte Harnstoffschmelze eine Fließgeschwindigkeit von 100-200 mal der des Harnstoff-Ammoniumsulfat-Eutektikums aus dem Verdampfer (15) hat und die Endmischung, die aus dem Mischen der hochkonzentrierten Harnstoffschmelze und des Harnstoff-Ammoniumsulfat-Eutektikums resultiert, weniger als 0,5 Gew.-% Ammoniumsulfat enthält; und wobei die niedrig konzentrierte Harnstofflösung eine Konzentration von weniger als 95 Gew.-% aufweist.

2. Verfahren nach Anspruch 1, bei dem die aus dem Wäscher (13) abgezogene Ammoniumsulfatlösung mit Schwefelsäure ergänzt wird, bevor sie dem Verdampfer (15) zugeführt wird.

3. Verfahren nach Anspruch 1 oder 2, wobei die dem Verdampfer (15) zugeführte Ammoniumsulfatlösung eine Konzentration von bis zu etwa 40 Gew.-% aufweist.

4. Verfahren nach einem der vorstehenden Ansprüche, wobei die niedrig konzentrierte Harnstofflösung eine Konzentration von weniger als 90 Gew.-% aufweist.

5. Verfahren nach einem der vorstehenden Ansprüche, umfassend einen Schritt des Konzentrierens der im Wäscher (13) gebildeten Ammoniumsulfatlösung und der niedrig konzentrierten Harnstofflösung, mit Abtrennung einer Dampfphase und Bildung des Harnstoff-Ammoniumsulfat-Eutektikums.

6. Verfahren nach einem der vorstehenden Ansprüche, wobei die Ammoniumsulfatlösung von dem Wäscher (13) eine Konzentration von bis zu etwa 40 Gew.-% und die in der Harnstoffanlage (1) hergestellte niedrig konzentrierte Harnstofflösung eine Konzentration von etwa 85 Gew.-% aufweist.

7. Verfahren nach einem der vorstehenden Ansprüche, wobei das Eutektikum eine Ammoniumsulfat-Konzentration von weniger als 60 Gew.-% aufweist.

8. Verfahren nach einem der vorstehenden Ansprüche, wobei die Verfestigungseinheit (10) einen Prillturm (11) oder einen Granulator (71) umfasst.

9. Verfahren nach einem der vorstehenden Ansprüche, umfassend die Schritte des Zuführens von Schwefelsäure in den Wäscher (13); und des Zurückführens eines Ausflusses, der die im Wäscher (13) erzeugte Ammoniumsulfatlösung enthält, in den Wäscher (13).

10. Verfahren nach Anspruch 9, bei dem ein Teil der Ammoniumsulfatlösung aus dem Wäscher (13) nach Zugabe von frischer Schwefelsäure wieder in den Wäscher (13) zurückgeführt und ein Teil dem Verdampfer (15) zugeführt wird.

11. Verfahren nach einem der vorstehenden Ansprüche, wobei der Verdampfer (15) ein Vakuumverdampfer ist.

12. Verfahren nach einem der vorstehenden Ansprüche, umfassend die folgenden Schritte:
Zuführen einer im Verdampfer (15) abgetrennten Dampfphase zu einem Kondensator (48); und Kondensieren der Dampfphase zur Bildung eines Kondensats, das, möglicherweise unter Zugabe von Wasser von außen, dem Wäscher (13) zugeführt wird.

13. Verfahren nach einem der vorstehenden Ansprüche, umfassend einen Schritt des Vorkonzentrierens der Ammoniumsulfatlösung aus dem Wäscher (13), bevor sie dem Verdampfer (15) zugeführt wird.

14. Verfahren nach Anspruch 13, umfassend einen Schritt des Mischens der konzentrierten Ammoniumsulfatlösung mit einem ersten Teil der niedrig konzentrierten Harnstofflösung, um eine konzentrierte wässrige Lösung zu bilden, die z.B. etwa 40-50 Gew.-% Ammoniumsulfat enthält und die dem Verdampfer (15) zugeführt wird, um das Wasser zu entfernen und das Harnstoff-Ammoniumsulfat-Eutektikum zu bilden.

## Revendications

1. Procédé pour récupérer du sulfate d'ammonium à partir d'un courant gazeux produit dans une usine d'urée (1), et en particulier par une unité de solidification (10) de l'usine d'urée, comprenant les étapes suivantes
- traitement d'un courant gazeux provenant d'une unité de solidification (10) dans un épurateur (13) connecté à l'unité de solidification (10), pour éliminer l'ammoniac dudit courant gazeux par réaction avec de l'acide sulfurique et avec formation d'une solution aqueuse de sulfate d'ammonium ;
- retrait de la solution de sulfate d'ammonium hors de l'épurateur (13) ;
- mélange de la solution de sulfate d'ammonium avec une solution d'urée faiblement concentrée produite dans l'usine d'urée (1), et élimination de l'eau dans un évaporateur (15), pour former un eutectique d'urée-sulfate d'ammonium ; l'eutectique ayant une concentration de sulfate d'ammonium de 30 à 60 % en poids, un point de fusion de 121 à 150°C et une teneur en eau inférieure à 1 % ;
- recirculation d'une partie dudit eutectique depuis l'évaporateur (15) jusqu'au sommet (36) de l'évaporateur (15), et mélange du reste avec une masse fondue d'urée fortement concentrée produite par l'usine d'urée (1) ;
- introduction dudit eutectique, mélangé avec ladite masse fondue d'urée fortement concentrée produite par l'usine d'urée (1), dans l'unité de solidification (10) ;
dans lequel la solution d'urée faiblement concentrée introduite dans l'évaporateur (15) a une concentration inférieure à celle de la masse fondue d'urée fortement concentrée mélangée avec l'eutectique ; et dans lequel la solution de sulfate d'ammonium formée dans l'épurateur (13) est introduite dans l'évaporateur (15) où l'eutectique d'urée-sulfate d'ammonium est formé ; la masse fondue d'urée fortement concentrée a un débit de 100 à 200 fois celui de l'eutectique d'urée-sulfate d'ammonium provenant de l'évaporateur (15) et le mélange final résultant du mélange de la masse fondue d'urée fortement concentrée et de l'eutectique d'urée-sulfate d'ammonium contient moins de 0,5 % en poids de sulfate d'ammonium ;
et dans lequel la solution d'urée faiblement concentrée a une concentration inférieure à 95 % en poids.

2. Procédé selon la revendication 1, dans lequel la solution de sulfate d'ammonium retirée de l'épurateur (13) est supplémentée avec de l'acide sulfurique avant d'être introduite dans l'évaporateur (15).

3. Procédé selon la revendication 1 ou 2, dans lequel la solution de sulfate d'ammonium introduite dans l'évaporateur (15) a une concentration allant en gros jusqu'à 40 % en poids.

4. Procédé selon l'une des revendications précédentes, dans lequel la solution d'urée faiblement concentrée a une concentration inférieure à 90 % en poids.

5. Procédé selon l'une des revendications précédentes, comprenant une étape de concentration de la solution de sulfate d'ammonium formée dans l'épurateur (13) et de la solution d'urée faiblement concentrée, avec séparation de la phase vapeur et formation de l'eutectique d'urée-sulfate d'ammonium.

6. Procédé selon l'une des revendications précédentes, dans lequel la solution de sulfate d'ammonium provenant de l'épurateur (13) a une concentration allant en gros jusqu'à 40 % en poids, et la solution d'urée faiblement concentrée produite dans l'usine d'urée (1) a une concentration en gros de 85 % en poids.

7. Procédé selon l'une des revendications précédentes, dans lequel l'eutectique a une concentration de sulfate d'ammonium inférieure à 60 % en poids.

8. Procédé selon l'une des revendications précédentes, dans lequel l'unité de solidification (10) comprend une tour de grelonage (11) ou un granulateur (71) .

9. Procédé selon l'une des revendications précédentes, comprenant les étapes d'introduction d'acide sulfurique dans l'épurateur (13) ; et de recirculation vers l'épurateur (13) d'un courant sortant contenant la solution de sulfate d'ammonium produite dans l'épurateur (13).

10. Procédé selon la revendication 9, dans lequel une partie de la solution de sulfate d'ammonium provenant de l'épurateur (13) est renvoyée en recirculation dans l'épurateur (13), après l'addition d'acide sulfurique frais, et une partie est introduite dans l'évaporateur (15) .

11. Procédé selon l'une des revendications précédentes, dans lequel l'évaporateur (15) est un évaporateur sous vide.

12. Procédé selon l'une des revendications précédentes, comprenant les étapes de : introduction d'une phase vapeur, séparée dans l'évaporateur (15), dans un condenseur (48) ; et condensation de la phase vapeur pour former un condensat qui, éventuellement avec l'addition d'eau depuis l'extérieur, est introduit dans l'épurateur (13).

13. Procédé selon l'une des revendications précédentes, comprenant une étape de pré-concentration de la solution de sulfate d'ammonium provenant de l'épurateur (13) avant qu'elle soit introduite dans l'évaporateur (15).

14. Procédé selon la revendication 13, comprenant une étape de mélange de la solution de sulfate d'ammonium concentrée avec une première partie de la solution d'urée faiblement concentrée, pour former une solution aqueuse concentrée contenant en gros 40 à 50 % en poids de sulfate d'ammonium, et qui est introduite dans l'évaporateur (15) pour que l'eau soit éliminée et pour former l'eutectique d'urée-sulfate d'ammonium.
